# EUROPÄISCHE PATENTANMELDUNG

(11) **EP 3 991 672 A2**
(43) Veröffentlichungstag der Anmeldung: **04.05.2022**
(21) Anmeldenummer: 21204786.4
(22) Anmeldetag: 26.10.2021
(51) Int. Cl.: A61B 17/68, A61B 17/86, A61B 17/88

(54) **MEDIZINISCHES VERSCHRAUBUNGSSYSTEM FÜR VERSORGUNGEN IM BEREICH DES BECKENS**

(30) Priorität: 27.10.2020 DE 102020128211
(71) Anmelder: Dieter Marquardt Medizintechnik GmbH, 78549 Spaichingen (DE)
(72) Erfinder: Dietrich, Timo, 78549 Spaichingen (DE); Verheyden, Akhil P., 79110 Freyburg (DE)
(74) Vertreter: Bobbert & Partner Patentanwälte PartmbB

(57) **Zusammenfassung**

Die vorliegende Erfindung betrifft ein medizinisches Verschraubungssystem (100) zum Einsatz im Bereich des knöchernen Beckens. Das medizinische Verschraubungssystem (100) weist wenigstens einen Stab (10) mit einem ersten Endabschnitt (11) und einem zweiten Endabschnitt (13) auf oder besteht hieraus. Der Stab (10) ist dabei durchgängig kanüliert und der erste Endabschnitt (11) weist einen ersten Gewindeabschnitt (111) auf oder besteht hieraus. Die vorliegende Erfindung betrifft ferner ein Instrumentenset (200), ein Behandlungsset und ein Verfahren zum Sichern einer Verriegelungsmutter (21).

## Beschreibung

Die vorliegende Erfindung betrifft ein medizinisches Verschraubungssystem gemäß Anspruch 1, ein Instrumentenset gemäß Anspruch 11, außerdem ein Behandlungsset gemäß Anspruch 14 und ein Verfahren gemäß Anspruch 15 bzw. gemäß jeweils der Oberbegriffe oder Gattungsbegriffe dieser Ansprüche.

Sind im Bereich des knöchernen Beckens Frakturen des Sakrums oder am Sakrum oder Dislokationen des Iliosakralgelenks zu versorgen mit der Notwendigkeit einer Fixation des posterioren Beckens, so kommen hierzu Verschraubungssysteme zum Einsatz.

Eine Aufgabe der vorliegenden Erfindung ist es, ein weiteres medizinisches Verschraubungssystem, ein zur Verwendung hiermit vorgesehenes Instrumentenset sowie ein Behandlungsset und ein Verfahren zum Sichern des medizinischen Verschraubungssystems anzugeben.

Die erfindungsgemäße Aufgabe wird durch ein medizinisches Verschraubungssystem mit den Merkmalen des Anspruchs 1 und durch ein Instrumentenset mit den Merkmalen des Anspruchs 11 gelöst. Zudem wird sie gelöst durch ein Behandlungsset mit den Merkmalen des Anspruchs 14 und durch das Verfahren mit den Merkmalen des Anspruchs 15.

Die vorliegende Erfindung betrifft ein medizinisches Verschraubungssystem (im Folgenden auch kurz:
Verschraubungssystem) zum Einsatz im Bereich des knöchernen Beckens, insbesondere bei Frakturen des oder am Sakrum, bei Dislokationen des Iliosakralgelenks mit der Notwendigkeit einer Fixation des posterioren Beckens.

Das erfindungsgemäße Verschraubungssystem weist wenigstens einen Stab zum Einsatz im Beckenbereich mit einem ersten Endabschnitt und einem zweiten Endabschnitt auf, oder besteht aus diesem Stab. Der Stab ist durchgängig, also entlang seiner gesamten Längserstreckung, kanüliert. Sein erster Endabschnitt weist einen ersten Gewindeabschnitt auf oder besteht hieraus.

Eine Kanülierung kann hierin grundsätzlich als Längskanal verstanden werden.

Die vorliegende Erfindung betrifft ferner ein Instrumentenset. Es umfasst oder besteht aus Instrumenten zum minimalinvasiven Einbringen eines medizinischen Verschraubungssystems zum Einsatz im Bereich des knöchernen Beckens, insbesondere bei Frakturen des oder am Sakrum, bei Dislokationen des Iliosakralgelenks mit der Notwendigkeit einer Fixation des posterioren Beckens, insbesondere eines erfindungsgemäßen Verschraubungssystems, alternativ eines Stabs jenes Verschraubungssystems.

Das erfindungsgemäße Instrumentenset weist ein erstes kanüliertes Drehwerkzeug zum Drehen oder Schrauben der Kompressionsmutter des medizinischen Verschraubungssystems auf. Die Kanülierung, die ein zumeist in Längsrichtung des betreffenden Gegenstands verlaufendes Lumen ergibt oder ist, ist sowohl in Durchmesser als auch Länge dimensioniert, um ein zweites Drehwerkzeug zum Drehen oder Schrauben der Verriegelungsmutter in sich aufzunehmen.

Ferner betrifft die vorliegende Erfindung ein Behandlungsset, aufweisend ein erfindungsgemäßes medizinisches Verschraubungssystem und ein erfindungsgemäßes Instrumentenset.

Das erfindungsgemäße Verfahren dient zum Sichern einer Kompressionsmutter, welche auf einen minimalinvasiv eingebrachten Stab eines erfindungsgemäßen medizinischen Verschraubungssystems auf dessen ersten Endabschnitt aufgesetzt ist oder wird und mittels einer Verriegelungsmutter fixiert wird.

Das Verfahren umfasst den Schritt eines Aufsetzens des ersten kanülierten Drehwerkzeugs des erfindungsgemäßen Instrumentensets auf die Kompressionsmutter, alternativ ein Halten des bereits aufgesetzten Drehwerkzeugs, sollte es bereits auf die Kompressionsmutter aufgesetzt sein, in seiner aufgesetzten Position.

Das Verfahren umfasst ferner ein Halten der Kompressionsmutter mittels des ersten kanülierten Drehwerkzeugs in seiner Ausgangsdrehposition oder -winkelposition, alternativ ein weiteres Verspannen desselben in einer ersten Drehrichtung.

Ferner umfasst das Verfahren als weiteren Schritt ein Aufsetzen des zweiten Drehwerkzeugs des erfindungsgemäßen Instrumentensets auf die Verriegelungsmutter, wobei oder während diese mittels Einschiebens des zweiten Drehwerkzeugs durch die Kanülierung des ersten Drehwerkzeugs am Stab oder an der Kompressionsmutter positioniert wird, oder wobei diese bereits zuvor auf den Stab oder gegen die Kompressionsmutter aufgeschraubt wurde.

Außerdem ist ein Drehen oder Schrauben der Verriegelungsmutter mittels des zweiten Drehwerkzeugs in einer der ersten Drehrichtung entgegengesetzten Drehrichtung gegen das erste Drehwerkzeug unter Kontern, oder zum Kontern, der Kompressionsmutter als weiterer Schritt vom erfindungsgemäßen Verfahren umfasst.

Alle mit den erfindungsgemäßen Verfahren erzielbaren Vorteile lassen sich in bestimmten erfindungsgemäßen Ausführungsformen ungeschmälert auch mit den erfindungsgemäßen Vorrichtungen erzielen, und umgekehrt.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche und Ausführungsformen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt diese eine erfindungsgemäße, beispielhafte Ausführungsform dar.

Wenn hierin offenbart ist, dass der erfindungsgemäße Gegenstand ein oder mehrere Merkmale in einer bestimmten Ausführungsform aufweist, so ist hierin jeweils auch offenbart, dass der erfindungsgemäße Gegenstand genau dieses oder diese Merkmale in anderen, ebenfalls erfindungsgemäßen Ausführungsformen ausdrücklich nicht aufweist, z. B. im Sinne eines Disclaimers. Für jede hierin genannte Ausführungsform gilt somit, dass die gegenteilige Ausführungsform, beispielsweise als Negation formuliert, ebenfalls offenbart ist.

Erfindungsgemäße Ausführungsformen können eines, mehrere oder alle der oben und/oder im Folgenden genannten Merkmale in jeder technisch möglichen Kombination aufweisen.

In manchen Ausführungsformen des erfindungsgemäßen Verschraubungssystems ist der erste Gewindeabschnitt ein metrisches, vorzugsweise zweigängiges oder mehrgängiges, Gewinde oder weist ein solches auf.

In exemplarischen Ausführungsformen weist das metrische Gewinde eine Schneide auf, ist also vorzugsweise selbstschneidend.

In einigen Ausführungsformen weist der zweite Endabschnitt einen zweiten Gewindeabschnitt auf oder besteht hieraus. Bei dem zweiten Gewindeabschnitt kann es sich um ein Innengewinde oder, bevorzugt, um ein Außengewinde handeln.

In manchen Ausführungsformen ist der zweite Gewindeabschnitt vorzugsweise ein Außengewinde, etwa ein selbstschneidendes Knochengewinde und/oder ein Kortikalisgewinde, oder weist ein solches auf.

In einigen Ausführungsformen des Verschraubungssystems weist der Stab zwischen dem ersten Endabschnitt und dem zweiten Endabschnitt einen Zwischenabschnitt auf, welcher auf seiner Außenseite kein Gewinde trägt. Der Außendurchmesser des Zwischenabschnitts ist vorzugsweise, beispielsweise um das 0,25-fache, geringer als der Außendurchmesser des zweiten Gewindes.

In manchen Ausführungsformen weist das Verschraubungssystem weiter wenigstens eine Verriegelungsmutter, vorzugsweise mit Innengewinde, auf. Diese kann dazu dienen, die Kompressionsmutter zu blockieren oder zu kontern. Sie kann dabei den Stab endseitig begrenzen oder sein Ende bilden.

In einigen Ausführungsformen weist das Verschraubungssystem weiter eine erste Unterlegscheibe auf.

In manchen Ausführungsformen weist das Verschraubungssystem weiter wenigstens eine Kompressionsmutter auf. Die Kompressionsmutter kann dazu vorgesehen sein, um den Stab endseitig gegen die knöchernen Strukturen des Beckens zu verspannen, oder hierzu beitragen.

In einigen Ausführungsformen sind die Kompressionsmutter und die erste Unterlegscheibe mittels eines Gelenkabschnitts beweglich miteinander verbunden. Die Verbindung kann dauerhaft sein. Sie kann dergestalt sein, dass sie nicht gelöst werden kann, ohne zerstörend zu wirken.

In manchen Ausführungsformen weist die Kompressionsmutter einen größeren Außendurchmesser als die Verriegelungsmutter auf. In anderen Ausführungsformen ist dies nicht der Fall oder umgekehrt.

In einigen Ausführungsformen weist das Verschraubungssystem weiter eine zweite Unterlegscheibe auf. Die zweite Unterlegscheibe kann vorgesehen sein, den Stab gegen die knöchernen Strukturen des Beckens zu verspannen, oder hierzu beitragen.

In manchen Ausführungsformen sind die erste Unterlegscheibe und/oder die zweite Unterlegscheibe relativ beweglich zum ersten bzw. zweiten Endabschnitt auf den Stab jeweils aufgesetzt oder aufsetzbar.

In einigen Ausführungsformen ist die zweite Unterlegscheibe in einer axialen Richtung beweglich zwischen dem zweiten Gewindeabschnitt und einem Kopfabschnitt des zweiten Endabschnitts oder des Stabs angeordnet. Dabei kann sie vom zweiten Gewindeabschnitt und dem Kopfabschnitt in axialer Richtung des Stabs begrenzt sein.

Alternativ oder ergänzend kann die zweite Unterlegscheibe in axialer Richtung einerseits durch den zweiten Gewindeabschnitt und andererseits durch den Kopfabschnitt formschlüssig begrenzt angeordnet sein.

Erste und/oder zweite Unterlegscheibe können jeweils eine Durchgangsöffnung zum Hindurchführen des Stabs durch sie aufweisen, wobei die Durchgangsöffnung einen Durchmesser, eine Dimension oder eine Öffnungsfläche aufweist, welche/-r mindestens 125 % des entsprechenden Werts des Stabs an der Stelle beträgt, an welcher die Unterlegscheibe auf den Stab zum dortigen Verbleib aufgesetzt ist.

Dies sorgt dafür, dass die Unterlegscheibe nach ihrem Montieren axial auf dem Kopfabschnitt des Stabes fixiert ist, aber dennoch rotieren kann und im Winkel beweglich bleibt.

Erste und/oder zweite Unterlegscheibe können jeweils eine Durchgangsöffnung aufweisen, die einen Durchmesser hat, welcher der Stabmitte zugeordnet ist, welcher geringer ist als ein Durchmesser, welcher zum Stabende zugeordnet ist.

Erste und/oder zweite Unterlegscheibe sind vorzugsweise derart ausgestaltet, dass sie relativ zur Längsachse des Stabs einen Winkel außerhalb eines Bereichs, welcher sich zwischen 75 und 105° erstreckt, einnehmen können.

Erste und/oder zweite Unterlegscheibe sind vorzugsweise derart ausgestaltet, dass sie relativ zur Senkrechten auf die Längsachse des Stabs einen Winkel von wenigstens 20° einnehmen können.

In manchen Ausführungsformen weist der zweite Endabschnitt eine stirnseitige Eingriffsöffnung oder einen Antrieb auf. Diese dient zum Eingreifen mit der Spitze oder dem Endabschnitt eines Drehwerkzeugs, z. B. der Klinge eines Schraubendrehers (Schlitz, Inbus, Mehrkant wie Vier- oder Sechskant, Kreuzschlitz, Torx, usw.).

In einigen Ausführungsformen weist das erfindungsgemäße Verschraubungssystem eine Vielzahl von Stäben (wenigstens zwei) auf, von welchen wenigstens zwei oder mehr unterschiedliche Längen haben, wobei der Längenunterschied zwischen den einzelnen Stäben bevorzugt 5 mm beträgt. Dabei kann der kürzeste Stab vorzugsweise 150 mm oder weniger, der längste vorzugsweise 250 mm oder mehr betragen. Es sind jedoch auch andere Längen von der vorliegenden Erfindung umfasst. Die Stäbe können jeweils Unterlegscheiben, Kompressionsmuttern und/oder Verriegelungsmuttern aufweisen.

In manchen Ausführungsformen weist das erfindungsgemäße Instrumentenset weiter ein zweites, vorzugsweise kanüliertes, Drehwerkzeug zum Drehen oder Schrauben der Verriegelungsmutter auf. Das zweite Drehwerkzeug ist vorzugsweise ausreichend lang, um, einmal in das erste Drehwerkzeug eingesteckt, zumindest bis in einen Endbereich der Kanülierung zu reichen.

In manchen Ausführungsformen weist das Instrumentenset ferner ein Längenbestimmungsinstrument auf, welches Markierungen trägt, welche eine Auswahl jenes Stabs, der für die entsprechenden Einsatz am geeignetsten ist, aus den im Verschraubungssystem enthaltenen Stäbe erleichtert, indem Längenbereiche markiert sind, für welche die Verwendung z. B. eines Stabs einer ersten Länge oder eines Stab einer zweiten Länge vorgeschlagen wird.

Misst der Chirurg also beispielsweise mittels des Längenbestimmungsinstruments entlang des Führungsdrahts die Strecke bis zur Gegenkortikalis, so kann er vom Längenbestimmungsinstrument einfach und direkt die präferierte Länge des zur Versorgung empfohlenen Stabs ablesen. Hierzu kann das Längenbestimmungsinstrument ganz oder teilweise kanüliert sein. Alternativ kann es eine Längsnut aufweisen.

Es ist von der vorliegenden Erfindung ebenfalls umfasst, dass das Längenbestimmungsinstrument ergänzend oder alternativ andere Markierungen, beispielsweise gängige Längeneinheiten, z. B eine Skala in Zentimeter oder Inch, aufweisen kann.

In einigen Ausführungsformen weist das Instrumentenset außerdem wenigstens einen Kirschnerdraht auf. Der Kirschnerdraht kann in Länge und Durchmesser auf seinen Einsatz im Beckenbereich, beispielsweise während einer Operation, abgestimmt sein. So kann er beispielsweise eine Länge von 300 mm und einen Durchmesser von 2,8 mm aufweisen.

In manchen Ausführungsformen weist das Instrumentenset ferner einen Spiralbohrer auf. Dieser ist vorzugsweise kanüliert.

In einigen Ausführungsformen weist das Instrumentenset ergänzend eine Austauschhülse auf.

In manchen Ausführungsformen können die Schritte des Verfahrens nacheinander, überlappend oder simultan abgearbeitet werden, in bestimmten Ausführungsformen teils nacheinander, teils überlappend und teils simultan.

In manchen Ausführungsformen ist das erfindungsgemäße Verschraubungssystem, das erfindungsgemäße Instrumentenset und/oder das erfindungsgemäße Behandlungsset steril.

In einigen Ausführungsformen weist das medizinische Verschraubungssystem vorzugsweise kein Element auf, welches zum dauerhaften Verbleib, ganz oder teilweise, in der durchgängigen Kanülierung des Stabs, oder zum Verschrauben darin, vorgesehen ist.

In manchen Ausführungsformen weist eines oder mehrere der Elemente des Verschraubungssystems Ti-6Al-4V (oder Ti64) auf oder besteht hieraus.

In einigen Ausführungsformen beträgt der Durchmesser des wenigstens einen Stabs zwischen 5 mm und 10 mm, bevorzugt 7,5 mm, die Länge zwischen 120 mm und 250 mm, weniger als 120 mm oder mehr als 250 mm.

In einigen Ausführungsformen weist das Verschraubungssystem oder der Stab keine Hülse, keine Hülse mit Kopf oder mit Linsenkopf auf.

In manchen Ausführungsformen hat der Stab keine Löcher in Querrichtung des Stabs und/oder keine radial abstehenden Finnen.

In einigen Ausführungsformen hat der Stab beidseits ein metrisches Außengewinde, sowie beidseits eine Kompressionsschraube und eine Verriegelungsschraube.

In manchen Ausführungsformen weist die Durchgangsöffnung der erste Unterlegscheibe und/oder der zweiten Unterlegscheibe einen zu ihrer Oberseite hingewandten Durchmesser auf, welcher kleiner ist als ein Durchmesser, welcher zu ihrer Unterseite hingewandt ist.

In einigen Ausführungsformen weist die Durchgangsöffnung der ersten Unterlegscheibe und/oder der zweiten Unterlegscheibe einen Durchmesser auf, welcher kleiner ist als ein maximaler Durchmesser des Kopfabschnitts des Stabs bzw. eines in die Unterlegscheibe eingeführten Gelenkabschnitt der Kompressionsmutter.

Auf diese Weise kann bewirkt werden, dass die erste und/oder die zweite Unterlegscheibe an der Kompressionsmutter bzw. am Kopfabschnitt des Stabes gehalten wird, aber dennoch rotieren kann und im Winkel zum Stab oder zur Längsrichtung der Kompressionsmutter kippen kann.

In einigen Ausführungsformen ist die Unterseite der ersten und/oder der zweiten Unterlegscheibe plan.

In manchen Ausführungsformen ist die Unterseite der ersten und/oder der zweiten Unterlegscheibe nicht in wenigstens zwei Ebenen schräg zueinander ausgebildet.

In einigen Ausführungsformen ist der Stab nicht zum Einschieben in eine Hülse oder eine andere Komponente vorgesehen, welche ihn über seinen größten Teil seiner Erstreckung bedecken würde und welche einen größeren Durchmesser als der Stab hat, bzw. eine solche Komponente ist nicht Teil des Verschraubungssystems.

In manchen Ausführungsformen ist der Stab kein zweiteiliges Element, dessen beide Teile ineinander eingesteckt sind, um den Stab teleskopierbar zu machen.

In einigen Ausführungsformen weist der Stab an seinem Kopfabschnitt keine Mutter und/oder kein Gewinde für eine Mutter auf.

In manchen Ausführungsformen weist das medizinische Verschraubungssystem weiter eine zweite Unterlegscheibe auf.

In einigen Ausführungsformen sind die erste Unterlegscheibe und/oder die zweite Unterlegscheibe relativ beweglich zum Stab auf den ersten bzw. zweiten Endabschnitt aufgesetzt oder aufsetzbar.

In manchen Ausführungsformen ist die zweite Unterlegscheibe in einer axialen Richtung beweglich zwischen dem zweiten Gewindeabschnitt und einem Kopfabschnitt des zweiten Endabschnitts und von zweitem Gewindeabschnitt und Kopfabschnitt in axialer Richtung des Stabs und/oder in axialer Richtung formschlüssig durch den zweiten Gewindeabschnitt einerseits und den Kopfabschnitt andererseits begrenzt angeordnet.

In einigen Ausführungsformen weist der zweite Endabschnitt eine stirnseitige Eingriffsöffnung zum Eingreifen mit der Spitze oder dem Endabschnitt eines Drehwerkzeugs, z. B. eines Schraubendrehers, auf.

In manchen Ausführungsformen weist das medizinisches Verschraubungssystem eine Vielzahl, wenigstens zwei, Stäbe unterschiedlicher Längen auf, wobei der Längenunterschied bevorzugt 5 mm beträgt.

In einigen Ausführungsformen weist das Instrumentenset weiter wenigstens einen Kirschnerdraht auf.

Manche oder alle erfindungsgemäßen Ausführungsformen können einen, mehrere oder alle der oben und/oder im Folgenden genannten Vorteile aufweisen.

Werden im Bereich des knöchernen Beckens, insbesondere bei Frakturen des Sakrums oder am Sakrum oder bei Dislokationen des Iliosakralgelenks mit der Notwendigkeit einer Fixation des posterioren Beckens, Verschraubungssysteme zur Fixierung verwendet, beinhalten diese massive Metallstäbe, welche mittels eines Bolzenschneiders auf die adäquate Länge gekürzt werden müssen. Dieses Verfahren macht einen minimalinvasiven Eingriff an dieser Stelle unmöglich. Dies ist erfindungsgemäß vorteilhafterweise anders: Es bedarf keiner solchen Kürzung, was es erlaubt, die erfindungsgemäßen Gegenstände zur minimalinvasiven Versorgung einzusetzen.

Die beweglich angeordneten Unterlegscheiben erlauben aufgrund ihrer Beweglichkeit relativ zum Stab, wozu auch ein Verkippen relativ zum Stab zählt, eine gleichmäßige Kraftverteilung am Knochen.

Das Vorsehen von unterschiedlich langen Stäben zur Auswahl durch den Chirurgen erlaubt vorteilhaft auch noch eine intraoperative Festlegung auf die optimale Endlänge, ohne dass das Implantat bzw. der Stab intraoperativ gekürzt werden muss. Dies eröffnet die Möglichkeit von minimalinvasiven Eingriffen durch das Verwenden eines der erfindungsgemäßen Systeme, insbesondere des Verschraubungssystems.

Der Zwischenabschnitt, der optional kein Gewinde trägt, kann zur Erhöhung der Festigkeit oder Dauerfestigkeit beitragen.

Das optionale Kortikalisgewinde kann zu einem erhöhten Halt des Implantats beitragen.

Die beweglich angeordneten oder hierfür vorbereiteten Unterlegscheiben können eine erhöhte tatsächliche Auflagefläche bieten und damit für eine gleichmäßigere Kraftverteilung um die Bohrung durch das Becken sorgen. Dies trägt vorteilhaft zum sicheren Sitz des Implantats bei.

Ein weiterer Vorteil kann sein, dass die Unterlegscheiben ein sicheres Entfernen bieten bzw. begünstigen können. Bei gewöhnlichen, am Markt befindlichen Unterlegscheiben, welche lose unter dem Schraubenkopf platziert werden ohne mit der zugehörigen Schaube verbunden zu sein, kann es vorkommen, dass die Unterlegscheibe mit dem Knochen oder Gewebe verwächst. Beim Explantieren des Stabs samt der hierzu zu lösenden Schraube kommt es daher oft vor, dass zwar die Schraube entfernt wird, die Unterlegscheibe jedoch an Ort und Stelle verbleibt und folglich eigens mit einer Pinzette oder Ähnlichem explantiert werden muss. Bei Verwendung der vorliegenden Erfindung wird aufgrund der festen Verbindung zwischen Unterlegscheibe und Stab die Unterlegscheibe vorteilhaft automatisch gemeinsam mit dem Stab bzw. mit der Kompressionsmutter zwangsexplantiert.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:
- **Fig. 1**: zeigt ein medizinisches Verschraubungssystem in einer ersten Ausführungsform mit einem ersten Stab;
- **Fig. 1a**: zeigt den ersten Stab der Fig. 1 ohne Kompressions- und Verriegelungsmutter;
- **Fig. 1b**: zeigt in vergrößerter, leicht perspektivischer Darstellung von der Seite die Kompressionsmutter und die erste Unterlegscheibe der Fig. 1;
- **Fig. 1c**: zeigt in vergrößerter, leicht perspektivischer Darstellung von der Seite die Verriegelungsmutter der Fig. 1;
- **Fig. 1d**: zeigt in vergrößerter Schnittdarstellung das Zusammenwirken von Kopfende eines Stabs des Verschraubungssystems in einer Ausführungsform und der Unterlegscheibe dieser Ausführungsform;
- **Fig. 2**: zeigt den ersten Stab und einen zweiten Stab des medizinischen Verschraubungssystems einer weiteren Ausführungsform in verschiedenen Längen;
- **Fig. 3a bis 3c**: zeigen ein erfindungsgemäßes Instrumentenset in einer ersten Ausführungsform; und

- **Fig. 4**: zeigt ein erstes Drehwerkzeug des Instrumentensets der Fig. 3, in welches ein zweites Drehwerkzeug des Instrumentensets eingeführt ist.

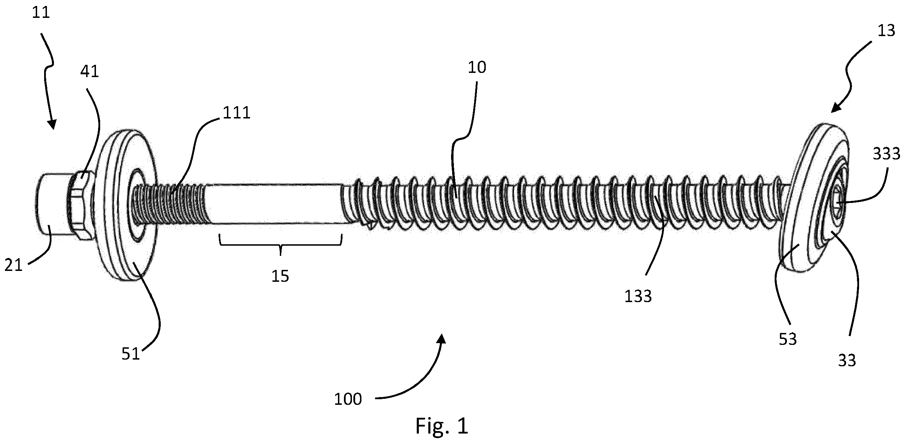

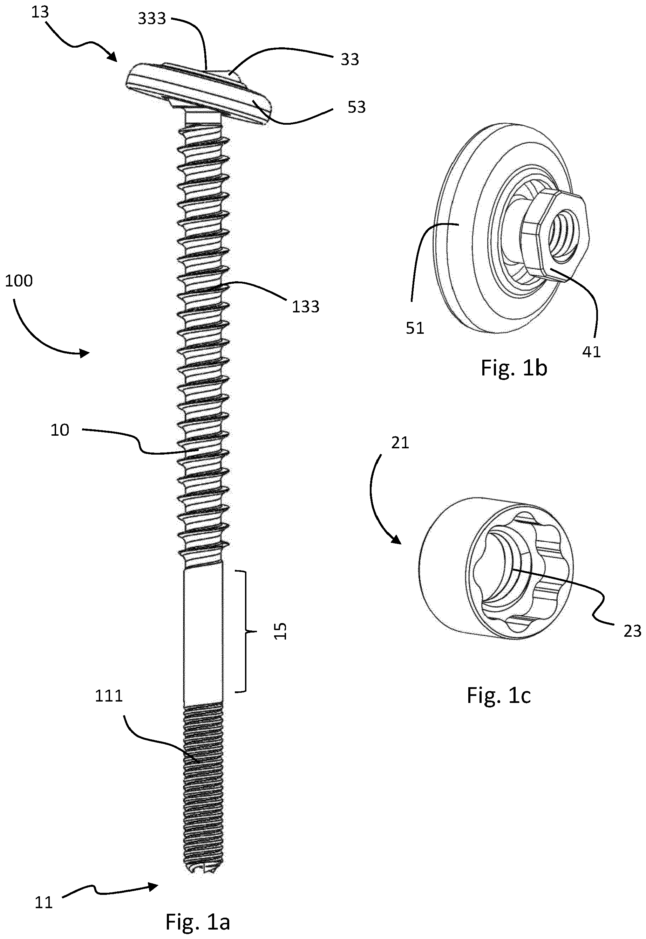

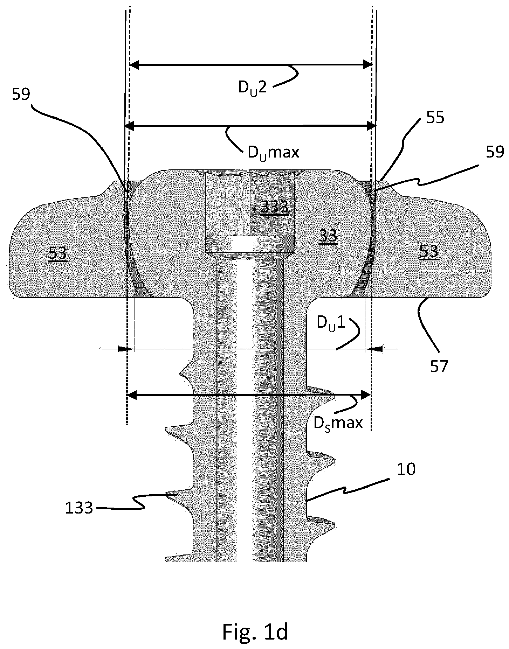

**Fig. 1** zeigt ein medizinisches Verschraubungssystem 100 (nachfolgend kurz: Verschraubungssystem) in einer ersten Ausführungsform zum Einsatz im Bereich des knöchernen Beckens in perspektivischer Ansicht. Es kann insbesondere bei Frakturen des Sakrums oder am Sakrum oder bei Dislokationen des Iliosakralgelenks mit Fixation des posterioren Beckens zum Einsatz kommen.

Das Verschraubungssystem 100 weist wenigstens einen Stab 10 mit einem ersten Endabschnitt 11 und einem zweiten Endabschnitt 13 auf oder besteht hieraus. Dabei ist der Stab 10 durchgängig, also entlang seiner gesamten Längserstreckung, kanüliert. Die Kanülierung erlaubt es, das Verschraubungssystem 100 minimalinvasiv, also bei fehlender oder äußerst beschränkter Blickkontrolle durch den Chirurgen, entlang eines zuvor eingebrachten Kirschnerdrahts und durch diesen geführt durch Gewebe und durch Knochenstrukturen (hier insbesondere Becken, Sakrum) vorzuschieben.

Der erste Endabschnitt 11 weist seinerseits einen ersten Gewindeabschnitt 111 auf oder besteht hieraus. Der erste Gewindeabschnitt 111 kann als Innengewinde oder, wie in Fig. 1, als Außengewinde ausgestaltet sein. Der Blick auf den ersten Gewindeabschnitt 111 ist in Fig. 1 weitgehend von einer optionalen, ersten Unterlegscheibe 51 und einer Kompressionsmutter 41, welche wie die erste Unterlegscheibe 51 Teil des Verschraubungssystems 100 sein kann, verdeckt.

Kompressionsmutter 41 und Unterlegscheibe 51 sind beide dem ersten Endabschnitt 11 zugeordnet und können insbesondere vorgesehen sein, den Stab 10 bzw. das Verschraubungssystem endseitig abzuschließen. Der erste Gewindeabschnitt 111 weist ein metrisches, vorzugsweise zwei- oder mehrgängiges, Gewinde auf oder besteht aus einem solchen. Das zumeist metrische Gewinde weist exemplarisch eine Schneide auf, ist also vorzugsweise selbstschneidend.

Die Kompressionsmutter 41 kann beweglich mit der Unterlegscheibe 51 verbunden sein, insbesondere gelenkig.

Der zweite Endabschnitt 13 weist einen optional zweiten Gewindeabschnitt 133 auf oder besteht hieraus. Der zweite Gewindeabschnitt 133 kann, vorzugsweise als Außengewinde, ein Kortikalisgewinde und/oder selbstschneidendes Knochengewinde sein oder ein solches aufweisen.

Zwischen dem ersten Endabschnitt 11 und dem zweiten Endabschnitt 13 weist der Stab 10 einen Zwischenabschnitt 15 auf, welcher auf seiner Außenseite vorzugsweise kein Gewinde trägt, und dessen Außendurchmesser vorzugsweise geringer als der Außendurchmesser des zweiten Gewindeabschnitts 133 ist.

Am ersten Endabschnitt 11 weist das Verschraubungssystem 100 weiter eine optionale Verriegelungsmutter 21 auf. Diese ist vorgesehen, um den Stab 10 bzw. das Verschraubungssystem 100 zu verspannen. Sie kann hierzu ein Innengewinde 23 aufweisen (siehe Fig. 1c). Ferner kann sie vorgesehen sein, den Endabschnitt endseitig abzuschließen, d. h. sein Ende zu bilden.

In bestimmten Ausführungsformen des Verschraubungssystems 100 sind die Kompressionsmutter 41 und die erste Unterlegscheibe 51 mit einem Gelenkabschnitt, der sie beweglich miteinander verbindet, verbunden. Optional sind sie unlösbar voneinander miteinander verbunden, jedenfalls vorzugsweise ohne zerstörend wirken zu müssen.

Es ist im Beispiel der Fig. 1 gut zu erkennen, dass die Kompressionsmutter 41 einen größeren Außendurchmesser als die Verriegelungsmutter 21 aufweist. Dies kann in manchen Ausführungsformen auch anders oder umgekehrt sein.

Das erfindungsgemäße Verschraubungssystem 100 weist in manchen Ausführungsformen wie der in Fig. 1 gezeigten weiter eine zweite Unterlegscheibe 53 auf. Diese kann dem zweiten Endabschnitt 13 zugeordnet und dazu vorgesehen sein, den Stab 10 bzw. das Verschraubungssystem 100 endseitig abzuschließen.

Die zweite Unterlegscheibe 53 kann eine Durchgangsöffnung für den zweiten Endabschnitt 13 aufweisen, welche einen Durchmesser, eine Dimension oder eine Öffnungsfläche aufweist, welche mindestens 125 % des entsprechenden Werts des Stabs an der Stelle beträgt, an welcher die Unterlegscheibe auf den Stab zum dortigen Verbleib aufgesetzt ist.

Die zweite Unterlegscheibe 53 ist in einer axialen Richtung beweglich zwischen dem zweiten Gewindeabschnitt 133 und einem Kopfabschnitt 33 des zweiten Endabschnitts 13 angeordnet. Hierbei wird ihre Bewegungsfreiheit in axialer Richtung vom zweiten Gewindeabschnitt 133 und vom Kopfabschnitt 33 des Stabs 10 und/oder formschlüssig durch den zweiten Gewindeabschnitt 133 einerseits und den Kopfabschnitt 33 andererseits begrenzt. Der Kopfabschnitt 33 kann während der Herstellung des Stabs 10 nach Auffädeln der zweiten Unterlegscheibe 53 auf den Stab 10 durch Stauchen des zweiten Endabschnitts 13 auf einen Durchmesser umgeformt worden sein, der ein Herunterrutschen der zweiten Unterlegscheibe 53 vom Stab 10 verhindert.

Der zweite Endabschnitt 13 weist vorzugsweise eine stirnseitige Eingriffsöffnung 333 (oder Antrieb) zum Eingreifen mit der Spitze eines Drehwerkzeugs, z. B. dem Ende eines Schraubendrehers, auf.

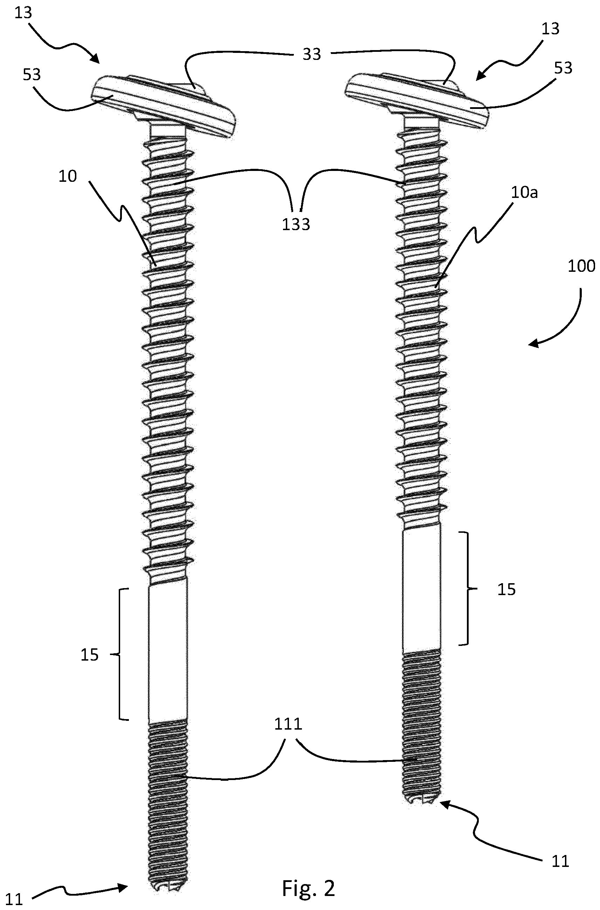

Das Verschraubungssystem 100 kann eine Vielzahl, wenigstens zwei, gemäß dem Beispiel in Fig. 1 ausgestalteten Stäbe 10, 10a in unterschiedlichen Längen aufweisen (siehe Fig. 2).

Es kann erfindungsgemäß vorgesehen sein, die zweite Unterlegscheibe 53 oder einen anderen Abschnitt des Stabs 10, 10a mit der Länge des entsprechenden Stabes zu kennzeichnen.

**Fig. 1a** zeigt den ersten Stab 10 der Fig. 1 ohne Kompressionsmutter 41 und ohne Verriegelungsmutter 21. Um Wiederholungen zu vermeiden wird auf die Beschreibung zu Fig. 1 verwiesen.

**Fig. 1b** zeigt in vergrößerter, leicht perspektivischer Darstellung die Kompressionsmutter 41 und die erste Unterlegscheibe 51 der Fig. 1.

Sowohl für den Stab 10 als auch für die Kompressionsmutter 41 kann dasselbe Bauteil als Unterlegscheibe 51, 53 verwendet werden.

**Fig. 1c** zeigt in vergrößerter, leicht perspektivischer Darstellung die Verriegelungsmutter 21 der Fig. 1.

Gut zu erkennen ist in Fig. 1c das Innengewinde 23 der Verriegelungsmutter 21. Dieses dient zum Verschrauben der Verriegelungsmutter 21 mit dem ersten Gewindeabschnitt 111, um die Kompressionsmutter 41 und damit den Stab 10, 10a sicher am bzw. im Becken zu fixieren.

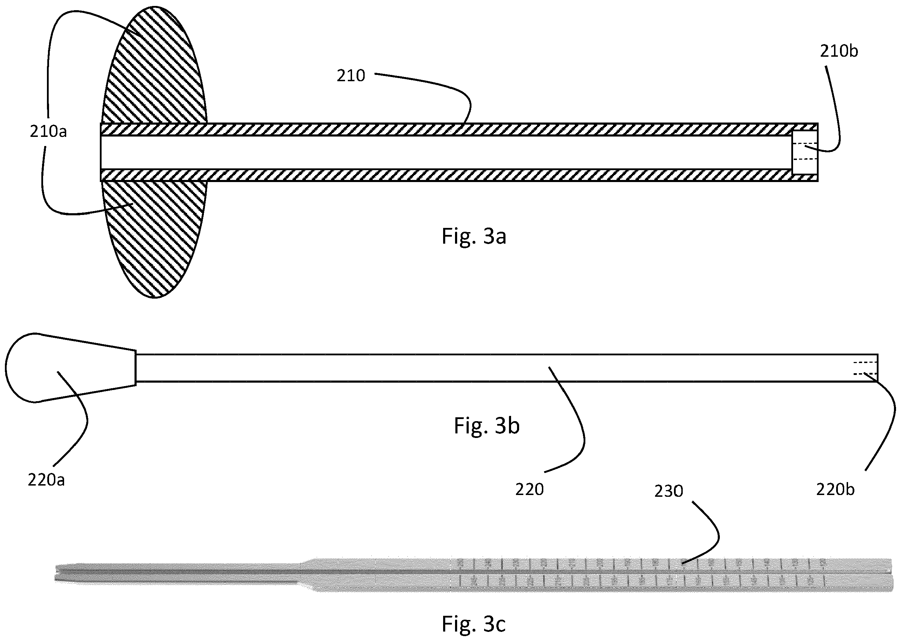

Ein Eingriff oder ein Antrieb für ein stirnseitiges Aufsetzen des Drehwerkzeugs 220, siehe exemplarisch Fig. 3b, ist erkennbar. Die Verriegelungsmutter 21 kann einen Torx-Eingriff oder einen anderen Eingriff, insbesondere zum Aufnehmen des Endabschnitts eines Drehwerkzeugs, aufweisen.

**Fig. 1d** zeigt in vergrößerter Schnittdarstellung das Zusammenwirken zwischen Kopfabschnitt 33 eines (nur abschnittsweise gezeigten) Stabs 10 des medizinischen Verschraubungssystems 100 in einer Ausführungsform und der zweiten Unterlegscheibe 53 dieser Ausführungsform.

Die zweite Unterlegscheibe 53 hat eine Oberseite 55 und eine Unterseite 57. Letztere ist der Mitte des Stabs 10 zugewandt, Erstere dem Ende des Stabs 10.

Die zweite Unterlegscheibe 53 ist auf den Kopfabschnitt 33 aufgesetzt bzw. der Kopfabschnitt 33 ist in die Durchgangsöffnung der Unterlegscheibe 53 ganz oder teilweise eingeführt.

Zu erkennen sind in Fig. 1d ein erster Durchmesser D_{U}1 und ein zweiter Durchmesser D_{U}2, wobei der erste Durchmesser D_{U}1 oberhalb (bezogen auf die Darstellung der Fig. 1d) des zweiten Durchmessers Du2 angeordnet ist.

Im konkreten Beispiel der Fig. 1d ist überdies erkennbar, dass der erste Durchmesser D_{U}1 die Durchgangsöffnung optional von der Unterseite 57 aus begrenzt, und dass der zweite Durchmessers D_{U}2 die Durchgangsöffnung optional auf der Oberseite 55 begrenzt bzw. deren Ende bildet.

Ist der erste Durchmesser D_{U}1, wie im Beispiel der Fig. 1d, geringer als der maximale Durchmesser Dsmax des Kopfabschnitts 33, so kann die zweite Unterlegscheibe 53 nicht über den Kopfabschnitt 33 hinweg und vom Stab 10 rutschen (in Fig. 1d nach oben).

Die hierfür erforderliche Verendung des einen Durchmessers der Unterlegscheibe auf den ersten Durchmesser D_{U}1 kann durch Verpressen oder anderes Verformen der zweiten Unterlegscheibe 53 herstellerseitig bewirkt worden sein.

Der zweite Durchmessers D_{U}2 der Durchgangsöffnung kann dem maximalen Durchmesser Dumax der Durchgangsöffnung entsprechen, dies ist jedoch optional.

Die Unterlegscheibe 53 kann am oberen Ende ihrer Durchgangsöffnung optional einen Vorsprung 59 oder eine Verformung aufweisen, welche geeignet ist, den Durchmesser der Durchgangsöffnung an diesem Ende so zu verkleinern, dass D_{U}2 < D_{U}max gilt. Damit kann vermieden werden, dass die Unterlegscheibe 53 über den Kopfabschnitt 33 hinweg und ggf. auch vom Stab 10 rutschen kann (in Fig. 1d nach unten).

Was mit Bezug auf Fig. 1d zur zweiten Unterlegscheibe 53 und dem Kopfabschnitt 33 ausgeführt ist, kann analog ebenso für die Verbindung zwischen der ersten Unterlegscheibe 51 und der Kompressionsmutter 41 zutreffen. Sie können ebenso durch die vorstehend beschriebene Wahl oder Erzeugung der Durchmesserverhältnisse aufeinander derart abgestimmt sein, dass sich die erste Unterlegscheibe 51 nicht von der Kompressionsmutter 41 lösen kann, aber dennoch rotieren kann und im Winkel beweglich bleibt, also kippen kann.

**Fig. 2** zeigt neben dem ersten Stab 10 einen zweiten Stab 10a. Sie unterscheiden sich hier in ihren verschiedenen Längen.

Die Ausgestaltung der Stäbe 10, 10a ist analog zu Fig. 1, allerdings ohne erste Unterlegscheibe 51, ohne Kompressionsmutter 41 und ohne Verriegelungsmutter 21. Um Wiederholungen zu vermeiden wird daher auf die Beschreibung der Fig. 1 verwiesen.

Das Verschraubungssystem 100 kann eine Vielzahl, wenigstens zwei, von Stäben 10, 10a unterschiedlicher Länge aufweisen, wobei der Längenunterschied bevorzugt 5 mm beträgt. Dabei kann der kürzeste Stab vorzugsweise 150 mm, der längste vorzugsweise 250 mm betragen. Es sind jedoch auch andere Längen von der vorliegenden Erfindung umfasst. Die Stäbe können jeweils endseitig abgeschlossen sein bzw. abgeschlossen werden.

Die Stäbe weisen vorzugsweise einen Durchmesser von 7,5 mm auf. Von der vorliegenden Erfindung sind größere oder kleinere Durchmesser ebenfalls umfasst.

**Fig. 3a** zeigt ein erstes Drehwerkzeug 210 als Bestandteil des erfindungsgemäßen Instrumentensets in einer Ausführungsform.

Das erste Drehwerkzeug 210 ist ausgestaltet bzw. dimensioniert, insbesondere kanüliert, um ein zweites Drehwerkzeug 220 (siehe Fig. 3b), vorzugsweise über seine gesamte Länge, darin aufzunehmen. Ferner ist es ausgestaltet, um mittels seines Klingenendes 210b die Kompressionsmutter 41 hinein- oder herausschrauben bzw. festziehen oder lösen zu können.

Am dem Klingenende 210b gegenüberliegenden Ende des ersten Drehwerkzeugs 210 ist ein Griffabschnitt 210a zum Halten oder Greifen des Drehwerkzeugs 210 vorgesehen.

**Fig. 3b** zeigt ein zweites Drehwerkzeug 220 als Bestandteil des erfindungsgemäßen Instrumentensets in einer Ausführungsform in Schnittdarstellung.

Das zweite Drehwerkzeug 220 ist derart ausgestaltet bzw. dimensioniert, um in ein erstes Drehwerkzeug 210 (siehe Fig. 3a) eingeführt werden zu können und um mit seinem Klingenende 220b die Verriegelungsmutter 21 hinein- oder herausschrauben bzw. festziehen oder lösen zu können. Es ist entsprechend lang ausgestaltet.

Das zweite Drehwerkzeug kann vorzugsweise kanüliert sein.

An jenem dem Klingenende 220b gegenüberliegenden Ende des zweiten Drehwerkzeugs 220 ist ein Griffabschnitt 220a vorgesehen, der wiederum dazu dient, das Drehwerkzeug 200a leichter greifen, halten oder mechanisch bedienen zu können bzw. dies zu ermöglichen.

**Fig. 3c** zeigt ein Längenbestimmungsinstrument 230 als optionalen Bestandteil eines erfindungsgemäßen Instrumentensets.

Das Längenbestimmungsinstrument 230 weist entlang seiner Längserstreckung Markierungen bzw. eine Skala auf. Die Markierungen bzw. die Skala können in manchen Ausführungsformen den Längen der im Verschraubungssystem 100 enthaltenen Stäbe 10, 10a entsprechen. Im Gebrauch kann das Längenbestimmungsinstrument 230 vorteilhaft die Länge eines zu bevorzugenden, zu verwendenden, insbesondere einzusetzenden, Stabes 10, 10a an- bzw. vorgeben im Sinne eines an den Chirurgen gerichteten Vorschlags.

In bestimmten Ausführungsformen umfasst das Instrumentenset 200 ferner wenigstens einen Kirschnerdraht (nicht in den Figuren gezeigt), abgestimmt in Länge und Durchmesser auf die Dimensionen im Einsatzgebiet der vorliegenden Erfindung, bevorzugt mit einem Durchmesser von 2,8 mm und einer Länge von z. B. 300 mm.

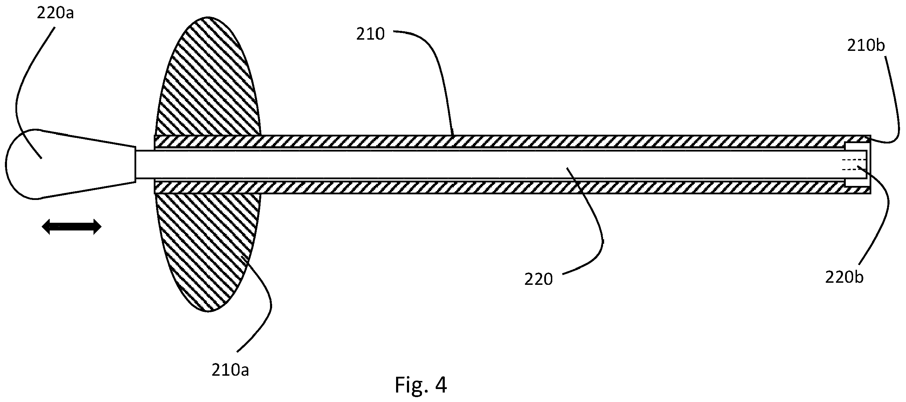

**Fig. 4** zeigt das erste Drehwerkzeug 210 des erfindungsgemäßen Instrumentensets der Fig. 3 mit eingeführtem zweiten Drehwerkzeug 220 des Instrumentensets.

Das kanülierte erste Drehwerkzeug 210 für die Kompressionsmutter 41 ist sowohl in Durchmesser als auch Länge derart dimensioniert, dass es das zweite Drehwerkzeug 220 für die Verriegelungsmutter 21 in seiner Kanülierung, zumindest bis in einen Endbereich der Kanülierung, aufnehmen kann. Dies ist in der Fig. 4 mittels eines Doppelpfeils angedeutet.

Insbesondere ist das erste Drehwerkzeug 210 ausgestaltet, um die Kompressionsmutter 41 festzuhalten und diese somit zu hindern mit der Verriegelungsmutter 21 mitzurotieren, wenn die Verriegelungsmutter 21 mittels des zweiten Drehwerkzeugs 220 festgezogen oder gelöst wird.

Ein typischer Einsatz der vorliegenden Erfindung kann wie folgt aussehen:
Der Zugang erfolgt durch eine Stichinzision über der geplanten Stabeintrittsstelle am Kreuzbein (auch: Sakrum, Höhe Wirbelkörper S1).

Der erste Sakralwirbelkörper S1 wird mit Hilfe eines Bildwandlers lokalisiert.

Der Kirschnerdraht wird im lateralen Strahlengang entsprechend den anatomischen Gegebenheiten bis zur gegenseitigen Kortikalis eingebracht.

Es folgen Inlet- und Outletaufnahmen und ggf. eine Korrektur der Drahtposition.

Die korrekte Position des Kirschnerdrahts wird vorzugsweise mittels eines 3D-Scans verifiziert. Der Kirschnerdraht sollte in einem Winkel von ca. 90° zum Frakturspalt verlaufen. Alternativ kann der Kirschnerdraht unter 3D-Navigation eingebracht werden.

Mit einem kanülierten Spiralbohrer, der Teil des erfindungsgemäßen Instrumentensets 200 sein kann, wird über den Kirschnerdraht durch die Bohrbüchse die Kortikalis aufgebohrt. Der Kirschnerdraht muss komplett überbohrt werden. Anschließend wird der überbohrte Kirschnerdraht entfernt.

Durch den im Körper verbliebenen kanülierten Spiralbohrer wird nun der Führungsdraht (z. B. Länge 520 mm) hindurchgeschoben. Eine Austauschhülse, welche ebenfalls Teil des erfindungsgemäßen Instrumentensets 200 sein kann, wird über den Führungsdraht bis auf Anschlag vorgeschoben. Der Führungsdraht muss bündig mit der Austauschhülse abschließen.

Anschließend wird das Längenbestimmungsinstrument 230 über den Führungsdraht bis zur Gegenkortikalis eingebracht. Es kann hierzu ganz oder teilweise kanüliert sein. Es kann eine Längsnut aufweisen.

Die benötigte Stablänge kann direkt auf der Skala des Längenbestimmungsinstruments 230 abgelesen werden. Das Längenbestimmungsinstrument 230 ist vorzugsweise zu einer Längsseite hin offen, damit der obere Teil, allerdings weniger als der volle Umfang, des Führungsdrahts sichtbar bleibt, um das Ende des Führungsdrahts mit der Skala auf dem Längenbestimmungsinstrument 230 vergleichen zu können. Sollte die so ermittelte Stablänge zwischen zwei Skalenwerten liegen wird vorzugsweise die längere Stablänge gewählt werden. Die Skala gibt somit Stablängen an, was nicht *cm* oder *inch* geschieht sondern in Zuordnungen zu verfügbaren Stäben.

Der Stab 10 des erfindungsgemäßen Verschraubungssystems 100 wird mit einem kanülierten Drehwerkzeug, wie einem Schraubendreher, über den Führungsdraht eingebracht.

Der Stab 10 muss soweit eingedreht werden, bis die Unterlegscheibe 53 des zweiten Endabschnitts 13 am Knochen anliegt.

Von der gegenüberliegenden Seite wird mittels des ersten Drehwerkzeugs 210 die Kompressionsmutter 41 mit Unterlegscheibe 51 über den Führungsdraht auf den ersten Endabschnitt 11 des Stabs 10 geschraubt und die gewünschte Kompression eingestellt.

Abschließend wird mit dem zweiten Drehwerkzeug 220 die Verriegelungsmutter 21 durch das erste Drehwerkzeug 210 hindurch eingebracht. Das zweite Drehwerkzeug 220 dringt mit seinem distalen Ende hierbei bis an das Ende der Kanülierung des ersten Drehwerkzeugs 210 vor. Unter Kontern der Kompressionsmutter 41 z. B. mittels Haltens des ersten Drehwerkzeugs 210, kann die Verriegelungsmutter 21 mittels des zweiten Drehwerkzeugs 220 handfest angezogen werden.

### Bezugszeichenliste

- 100: medizinisches Verschraubungssystem
- 10: Stab
- 10a: Stab
- 11: erster Endabschnitt
- 13: zweiter Endabschnitt
- 15: Zwischenabschnitt
- 21: Verriegelungsmutter
- 23: Innengewinde der Verriegelungsmutter
- 33: Kopfabschnitt
- 333: Eingriffsöffnung
- 41: Kompressionsmutter
- 51: erste Unterlegscheibe
- 53: zweite Unterlegscheibe
- 55: Oberseite der zweiten Unterlegscheibe
- 57: Unterseite der zweiten Unterlegscheibe
- 59: Vorsprung; Verformung
- 111: erster Gewindeabschnitt
- 133: zweiter Gewindeabschnitt
- 200: Instrumentenset
- 210: erstes Drehwerkzeug (für Kompressionsmutter)
- 210a: Griffabschnitt des ersten Drehwerkzeugs
- 210b: Klingenende des ersten Drehwerkzeugs
- 220: zweites Drehwerkzeug (für Verriegelungsmutter)
- 220a: Griffabschnitt des zweiten Drehwerkzeugs
- 220b: Klingenende des zweiten Drehwerkzeugs
- 230: Längenbestimmungsinstrument
- D_{U}1: erster Durchmesser Durchgangsöffnung Unterlegscheibe (Unterseite)
- D_{U}2: zweiter Durchmesser Durchgangsöffnung Unterlegscheibe (Oberseite)
- D_{U}max: maximaler Durchmesser Durchgangsöffnung Unterlegscheibe
- D_{S}max: maximaler Durchmesser Stab (Kopfabschnitt)

## Patentansprüche

1. Medizinisches Verschraubungssystem (100) zum Einsatz im Bereich des knöchernen Beckens, aufweisend oder bestehend aus:
- wenigstens einen Stab (10) mit einem ersten Endabschnitt (11) und einem zweiten Endabschnitt (13);
wobei der Stab (10) durchgängig kanüliert ist; und
wobei der erste Endabschnitt (11) einen ersten Gewindeabschnitt (111) aufweist oder hieraus besteht.

2. Medizinisches Verschraubungssystem (100) nach Anspruch 1, wobei der erste Gewindeabschnitt (111) ein metrisches, vorzugsweise zweigängiges, Gewinde, ist oder aufweist.

3. Medizinisches Verschraubungssystem (100) nach einem der vorangegangenen Ansprüche, wobei der zweite Endabschnitt (13) einen zweiten Gewindeabschnitt (133) aufweist oder hieraus besteht.

4. Medizinisches Verschraubungssystem (100) nach Anspruch 3, wobei der zweite Gewindeabschnitt (133) ein Kortikalisgewinde oder selbstschneidendes Knochengewinde ist oder aufweist.

5. Medizinisches Verschraubungssystem (100) nach einem der vorangegangenen Ansprüche, wobei der Stab (10) zwischen dem ersten Endabschnitt (11) und dem zweiten Endabschnitt (13) einen Zwischenabschnitt (15) aufweist, welcher auf seiner Außenseite kein Gewinde trägt, und dessen Außendurchmesser vorzugsweise geringer als der Außendurchmesser des zweiten Gewindes ist.

6. Medizinisches Verschraubungssystem (100) nach einem der vorangegangenen Ansprüche, weiter aufweisend wenigstens eine Verriegelungsmutter (21).

7. Medizinisches Verschraubungssystem (100) nach einem der vorangegangenen Ansprüche, weiter aufweisend eine erste Unterlegscheibe (51).

8. Medizinisches Verschraubungssystem (100) nach einem der vorangegangenen Ansprüche, weiter aufweisend wenigstens eine Kompressionsmutter (41).

9. Medizinisches Verschraubungssystem (100) nach einem der vorangegangenen Ansprüche, wobei die Kompressionsmutter (41) und die erste
Unterlegscheibe (51) mit einem Gelenkabschnitt, der sie beweglich miteinander verbindet, verbunden sind.

10. Medizinisches Verschraubungssystem (100) nach einem der vorangegangenen Ansprüche, wobei die Kompressionsmutter (41) einen größeren Außendurchmesser als die Verriegelungsmutter (21) aufweist.

11. Instrumentenset (200) mit Instrumenten zum minimalinvasiven Einbringen eines medizinischen Verschraubungssystems (100) zum Einsatz im Bereich des knöchernen Beckens, insbesondere nach einem der vorangegangenen Ansprüche, oder eines Stabs (10) dieses Verschraubungssystems (100), aufweisend:
- ein erstes kanüliertes Drehwerkzeug (210) für die Kompressionsmutter (41), wobei die Kanülierung sowohl in Durchmesser als auch Länge derart dimensioniert ist, um ein zweites Drehwerkzeug (220) für die Verriegelungsmutter (21) in sich aufzunehmen.

12. Instrumentenset (200) nach Anspruch 11, weiter aufweisend ein zweites, vorzugsweise kanüliertes, Drehwerkzeug (220) für die Verriegelungsmutter (21) zum Einschieben oder Einstecken in die Kanülierung des ersten Drehwerkzeugs (210), um vorzugsweise im eingesteckten Zustand bis zumindest in einen Endbereich der Kanülierung des ersten Drehwerkzeugs (210) zu reichen.

13. Instrumentenset (200) nach Anspruch 11 oder 12, weiter aufweisend ein Längenbestimmungsinstrument (230) mit markierten Längen entsprechend der Längen der im Verschraubungssystem (100) enthaltenen Stäbe (10, 10a).

14. Behandlungsset, aufweisend ein medizinisches Verschraubungssystem (100) nach einem der Ansprüche 1 bis 10 und ein Instrumentenset (200) nach einem der Ansprüche 11 bis 13.

15. Verfahren zum Sichern einer auf einen minimalinvasiv eingebrachten Stab (10) eines medizinisches Verschraubungssystem (100) nach einem der Ansprüche 1 bis 10 auf dessen ersten Endabschnitt (11) aufgesetzten Kompressionsmutter (41) mittels einer Verriegelungsmutter (21), mit den folgenden Schritten:
- Aufsetzen des ersten kanülierten Drehwerkzeugs (210) des Instrumentensets (200) nach einem der Ansprüche 11 bis 13 auf die Kompressionsmutter (41) oder Halten des bereits aufgesetzten Drehwerkzeugs (210) in seiner aufgesetzten Position;
- Halten der Kompressionsmutter (41) mittels des ersten kanülierten Drehwerkzeugs (210) in seiner Ausgangsdrehposition oder ein weiteres Verspannen desselben in einer ersten Drehrichtung;
- Aufsetzen des zweiten Drehwerkzeugs (220) des Instrumentensets (200) nach einem der Ansprüche 12 bis 13 auf die Verriegelungsmutter (21), wobei diese mittels Einschiebens des zweiten Drehwerkzeugs (220) durch die Kanülierung des ersten Drehwerkzeugs (210) am Stab (10) oder an der Kompressionsmutter (41) positioniert wird, oder wobei diese bereits zuvor auf den Stab (10) oder gegen die Kompressionsmutter (41) aufgeschraubt wurde; und
- Drehen oder Schrauben der Verriegelungsmutter (21) mittels des zweiten Drehwerkzeugs (220) in einer der ersten Drehrichtung entgegengesetzten Drehrichtung gegen das erste Drehwerkzeug (210) unter Kontern oder zum Kontern der Kompressionsmutter (41).
